(19) Europäisches Patentamt

European Patent Office

Office européen des brevets

(11) EP 0 565 448 B1

(12) EUROPEAN PATENT SPECIFICATION

(45) Date of publication and mention
of the grant of the patent:
**09.09.1998 Bulletin 1998/37**

(51) Int. Cl.$^6$: **A61M 25/00**, A61L 29/00,
A61M 25/10, A61M 29/02

(21) Application number: **93400917.6**

(22) Date of filing: **08.04.1993**

(54) **Catheter shaft**

Katheterschaft

Tige de cathéter

(84) Designated Contracting States:
**BE DE FR GB IT NL SE**

(30) Priority: **08.04.1992 JP 86846/92**
**28.04.1992 JP 110462/92**

(43) Date of publication of application:
**13.10.1993 Bulletin 1993/41**

(73) Proprietor:
**TERUMO KABUSHIKI KAISHA**
**Tokyo (JP)**

(72) Inventors:
 • **Kaneko, Takashi,**
 **c/o Terumo Kabushiki Kaisha**
 **Ashigarakami-gun, Kanagawa-ken (JP)**
 • **Mochizuki, Akira,**
 **c/o Terumo Kabushiki Kaisha**
 **Ashigarakami-gun, Kanagawa-ken (JP)**
 • **Kondo, Hiroshi,**
 **c/o Terumo Kabushiki Kaisha**
 **Ashigarakami-gun, Kanagawa-ken (JP)**
 • **Sagae, Kyuta,**
 **c/o Terumo Kabushiki Kaisha**
 **Ashigarakami-gun, Kanagawa-ken (JP)**

(74) Representative:
 **Gillard, Marie-Louise et al**
 **Cabinet Beau de Loménie**
 **158, rue de l'Université**
 **75340 Paris Cédex 07 (FR)**

(56) References cited:
 **EP-A- 0 274 411**   **EP-A- 0 349 640**
 **EP-A- 0 355 937**   **EP-A- 0 362 826**
 **EP-A- 0 417 552**   **GB-A- 2 153 687**

## Description

This invention relates to a catheter shaft which has an adequate flexibility as well as a sufficient mechanical strength, and which can be surface-treated to impart biocompatibility. This invention also relates to a shaft for a blood vessel-dilating catheter.

A catheter is a device which is passed through a tubular organ for injecting or evacuating a medical composition, a body fluid, or the like into or from a body cavity. Recently, there have been developed various types of highly functional catheters including a blood vessel-dilating catheter which is used for percutaneous transluminal coronary angioplasty (PTCA) to dilate the constricted portion of the blood vessel, and a urine balloon catheter equipped with a temperature sensor which can simultaneously withdraw urine from, and measure the temperature of, the bladder. In particular, balloon catheters which have a balloon on their tip portion are used for blood vessels and many other body cavities, and their significance in the medical field is increasing.

Of the balloon catheters, blood vessel dilatation catheters are used in percutaneous transluminal coronary angioplasty (PTCA) to dilate a stenosis, for example, in the coronary artery. In PTCA, a guiding catheter is first introduced into the femoral artery by using such means as the Serdinger method and passed through the blood vessel to the vicinity of the stenosis which is the target lesion by handling a guide wire, and then, a blood vessel dilatation catheter is inserted into and passed through the lumen of the guiding catheter until the tip balloon is located within the stenosis so that a dilatation fluid such as a contrast medium can be subsequently introduced into the balloon through the lumen within the dilatation catheter to thereby inflate the balloon and dilate or distend the stenosis.

Such a blood vessel-dilatation catheter is required to have trackability so that the dilatation catheter can smoothly pass through the guiding catheter along the winding blood vessel to the target lesion. The shaft of the dilatation catheter, therefore, must have sufficient strength as well as flexibility which is adequate for the tissue or the site to which the catheter is applied.

Furthermore, since the balloon at the tip of the blood vessel-dilatation catheter is inflated by applying pressure through the lumen of the catheter, the shaft portion of the catheter is required to have sufficient mechanical strength, in particular, sufficient pressure resistance to endure the pressure applied thereto. The pressure applied is generally about 7 atmospheres. Some operators, however, apply a pressure of about 14 atmospheres, or even higher. On the other hand, the catheter, which passes through the blood vessel, should have a diameter smaller than the diameter of the blood vessel. The inner diameter of the coronary artery at the constricted portion is about 3 mm at most, and therefore, the catheter shaft should have an exterior diameter of up to about 1.5 mm. While the exterior diameter is as small as up to 1.5 mm, the catheter shaft should have a lumen whose diameter should be sufficiently large to reduce flow resistance of the dilatation fluid flowing therethrough. The wall of the catheter shaft, therefore, inevitably becomes thin. Only a resin having an excellent mechanical strength can fulfill such a severe requirement and resist such a high pressure.

The catheter, which is introduced into the body cavity, should also be biocompatible. More illustratively, the catheter should have low abrasiveness, namely, high lubricity upon wetting by saliva or other digestion liquid, blood, or other body fluid, or physiological saline, water, or other aqueous liquid in respiratory tract, trachea, digestive tract, urinary tract, blood vessel, or other tissue. If the catheter shaft is insufficient in surface lubricity, the catheter cannot be smoothly inserted, and may injure the tissue into which the catheter is inserted to induce inflammation of the tissue. To avoid such injury and inflammation of the mucous membrane or other tissues to thereby reduce the pain of the patient, the surface of the catheter shaft should at least become lubricous upon wetting.

A blood vessel dilatation catheter, which is inserted into a blood vessel, should also have high anti-thrombotic properties.

Such properties are imparted with the catheter by surface modification, and therefore, the material used for fabricating the catheter shaft should be highly reactive to ensure sufficient adaptability to such surface modification.

Other properties required for the catheter shaft include high safety, sufficient sterilization resistance, fair workability, low production cost, and good storage stability.

Typical materials used for fabricating a shaft or tube portion of a catheter include polyethylenes, fluororesins, polyvinyl chlorides, and polyurethanes.

Plasticized, non-rigid polyvinyl chlorides have been a popular material in fabricating a catheter shaft for their adequate flexibility, heat resistance, workability, low production cost, high adaptability to surface modification, and the like, and in particular, for their ease in controlling the physical properties, especially flexibility, by simply adjusting the amount of the plasticizing agent added thereto. The plasticized, non-rigid polyvinyl chlorides, however, suffer from inevitable elusion of the hazardous plasticizing agent as well as poor mechanical strength due to the plasticizing agent added thereto in an amount as high as 30 to 70% by weight of the material.

Polyurethanes are also used in fabricating the catheter shaft. Polyurethane, however, is an expensive material, and suffers from degradation in the body.

Fluororesins and polyethylenes are also used for fabricating catheters because of their flexibility. Control of flexibility, however, is difficult, and even the most flexible commercially available polyester is insufficient in its flexibility for some types of catheters. The fluororesins and polyethylenes also suffer from poor surface lubricity upon wetting, and therefore, these materials should be surface-treated to render them hydrophilic. However, the fluororesins and polyethylenes are the materials which are known to be chemically quite stable, and which are most incompatible with surface modification by such means as plasma-initiated polymerization. The fluororesins and polyethylenes also suffer from poor adhesion with other resins or lubricants. Therefore, the catheter shafts fabricated from such materials suffer from difficulty in assembling with other components of the catheter, and even when the catheter shaft is coated with a hydrophilic polymer, it would be difficult to retain the effect of the surface coating for a prolonged period.

With regard to the blood vessel dilatation catheter, PCT/JP88/00202 and US-A-4,413,989 disclose the use of polyethylenes, polypropylenes, ethylene-propylene copolymers, ethylene-vinyl acetate copolymers, polyvinyl chlorides, polyamide elastomers, polyesters, polyurethanes and other thermoplastic resins, fluororesins, silicone rubbers, latex rubbers, and the like. Among the materials disclosed therein, the only material, however, which fulfilled the requirements in flexibility and strength and which could be used in practice was polyethylene.

EP-A-0 417 552 relates to a process intended to strenghten or stabilize prefabricated semifinished products or prefabricated elements made of an unsaturated polymer or a polymer alloy which contains at least an unsaturated polymer, wherein the parts to be crosslinked and/or sterilized are subjected to strong irradiation, characterized in that the unsaturated polymer is the 1,2-polybutadiene and that the parts are irradiated up to a value of 80 kGy. Particularly, a polymeric material containing an "olefin" compound in the form of a polybutadiene may be used in an angiographic catheter.

As mentioned above, the materials which are used for the shaft portion of a blood vessel dilatation catheter should have sufficient flexibility. In the case of polyethylene, an increase in flexibility is likely to be accompanied with a decrease in strength. The only material which could so far resist the pressure of 1.42 MPa (14 atmospheres), or even higher, while retaining its flexibility has been a high-density polyethylene.

In view of such a situation, in the conventional blood vessel dilatation catheter, emphasis has been laid on the improvement in trackability and the flexibility of the catheter shaft. For example, US-A-4,976,690 discloses a catheter shaft comprising a polyethylene material wherein opposite ends are tapered to impart the end portions with sufficient flexibility. US-A-4,775,371 discloses a catheter shaft wherein the end portion of the catheter shaft is tapered and overlaid with a polyethylene resin having higher flexibility to impart the end portion of the catheter shaft with sufficient flexibility. In such prior art catheter shafts, emphasis is basically laid on their mechanical strength and their ends are tapered to suppress their rigidity and impart flexibility, and no consideration whatsoever has been made for the surface treatment of the catheter shaft.

In view of the above-described situation, there is a strong demand for a catheter shaft material which may be readily surface-treated to impart lubricity and antithrombotic properties with the shaft surface, and whose flexibility may be controlled without adding any plasticizing agent and without detracting from the mechanical strength.

## SUMMARY OF THE INVENTION

An object of the present invention is to provide a catheter shaft which may be used for various body cavities, and in particular, a blood vessel. Another object of the invention is to provide a highly safe catheter shaft which may be readily surface-treated to impart lubricity and antithrombotic properties with the surface, and whose flexibility may be controlled without detracting from the mechanical strength.

According to the present invention, there is provided a catheter shaft of which at least a part is fabricated from a polymeric material comprising an $\alpha$-olefin polymer, a polymer alloy of two or more $\alpha$-olefin polymers or a polymer alloy of at least 10% by weight of an $\alpha$-olefin polymer with another polymer, said catheter shaft having a tensile modulus of up to 1,570 MPa (16,000 kg/cm$^2$) and a pressure-resisting strength of at least 6.2 MPa (63 kg/cm$^2$). Preferably the tensile modulus of the catheter shaft is in the range of from 138 to 1,570 MPa (1,400 to 16,000 kg/cm$^2$).

According to another aspect of the invention, there is provided a catheter shaft of which at least a part is fabricated from a polymeric material comprising at least one polymer of an $\alpha$-olefin having four or more carbon atoms, preferably in the range of from 4 to 7 carbon atoms, or a polymer alloy of at least 10% by weight of said at least one $\alpha$-olefin polymer with another polymer, said catheter shaft having a tensile modulus of up to 1,570 MPa (16,000 kg/cm$^2$) and a pressure-resisting strength of at least 6.2 MPa (63 kg/cm$^2$).

Preferably, the $\alpha$-olefin polymer has a tensile modulus of up to 491 MPa (5,000 kg/cm$^2$), preferably in the range of from 5.9 to 4,910 MPa (60 to 5,000 kg/cm$^2$) and is at least one member selected from the group consisting of polybutene, 1,2-polybutadiene, polyisoprene, polyisobutylene, propylenebutene copolymers, and copolymers of poly-4-methyl-1-pentene, among which polybutene-1 and 1,2-polybutadiene are preferred.

The alloying polymer may preferably be at least one member selected from the group consisting of polyethylene, ethylene-vinyl acetate copolymers, ethylene-vinyl chloride copolymers, polypropylene, ethylene-propylene copolymers, and propylene-butene copolymers, among which polypropylene and polyethylene are preferred.

The polymer alloy may preferably comprise a polymer blend of polybutene-1 and polypropylene.

When the catheter shaft is the shaft of a blood vessel-dilating catheter, it may preferably have a pressure-resisting strength of at least 11.1 MPa (113 kg/cm$^2$).

The polymer material used in the fabrication of the catheter shaft according to the invention is advantageously surface-modified to impart the material with biocompatibility.

Such surface modification may result from plasma treatment followed by coating with a maleic anhydride-based polymeric composition or from plasma-initiated graft polymerization with an acrylate compound.

DETAILED DESCRIPTION OF THE INVENTION

The catheter shaft of the present invention is fabricated from a polymeric material which comprises an $\alpha$-olefin polymer, a polymer alloy of two or more $\alpha$-olefin polymers, or a polymer alloy of at least 10% by weight of an $\alpha$-olefin polymer with another polymer.

An $\alpha$-olefin polymer is a high molecular weight hydrocarbon free from heteroatoms prepared by polymerizing an $\alpha$-olefin having a double bond at its terminal $\alpha$ site, which may have a side chain of 1 or more carbon atoms. The $\alpha$-olefin polymer may not necessarily be a homopolymer of an $\alpha$-olefin, but may also be a copolymer of two or more $\alpha$-olefins or a copolymer of an $\alpha$-olefin with another monomer. Exemplary $\alpha$-olefins include propylene, 1-butene, butadiene, 4-methyl-1-pentene, 1-pentene, and 1-hexene.

An $\alpha$-olefin polymer has a tertiary carbon-hydrogen bond, which may easily become cleaved upon irradiation of the polymer with plasma or radiation to form a radical. Therefore, the polymeric material used for preparing the catheter shaft of the present invention, which comprises a sufficient amount of such an $\alpha$-olefin polymer, (preferably at least 10% by weight, more preferably at least 20% by weight), may be readily surface-modified to impart the desired surface properties to the material.

The $\alpha$-olefin polymer used for preparing the polymer alloy of the present invention may preferably have a tensile modulus in the range of from 15.7 to 491 MPa (160 to 5,000 kg/cm$^2$).

Exemplary $\alpha$-olefin polymers having a tensile modulus in the range of from 15.7 to 491 MPa (160 to 5,000 kg/cm$^2$) include polybutene, 1,2-polybutadiene, polyisoprene, polyisobutylene, propylene-butene copolymers, and copolymers of poly-4-methyl-1-pentene, among which polybutene-1 and 1,2-polybutadiene are preferred for their commercial availability, good compatibility with the alloying polymer, and ease in controlling the flexibility of the polymer alloy.

The polymer alloyed with the $\alpha$-olefin polymer which preferably has a tensile modulus in the range of from 15.7 to 491 MPa (160 to 5,000 kg/cm$^2$) may be any desired polymer so long as the resulting polymer alloy has a tensile modulus of up to 1,570 MPa (16,000 kg/cm$^2$) and a pressure-resisting strength of at least 6.2 MPa (63 kg/cm$^2$). Exemplary alloying polymers include polyethylene, ethylene-vinyl acetate copolymers, ethylene-vinyl chloride copolymers, polypropylene, ethylene-propylene copolymers, and propylene-butene copolymers, among which polypropylene and polyethylene are preferred.

The polymer alloy may preferably comprise a polymer blend of polybutene-1 or 1,2-polybutadiene and polypropylene or polyethylene, and more preferably a polymer blend of polybutene-1 in view of good flexibility and mechanical strength, most preferably a polymer blend of polybutene-1 and polypropylene in view of good compatibility, good mechanical strength, adaptability to surface treatment, and workability.

The ratio of the $\alpha$-olefin polymer to the alloying polymer is not particularly limited so long as the resulting polymer alloy has the predetermined physical properties. However, in order to provide the catheter shaft material with sufficient adaptability to surface treatment, the $\alpha$-olefin polymer should comprise at least 10% by weight of the polymer material, and preferably, at least 20% by weight of the polymer material.

The polymer material is prepared, for example, by charging the weighed resins into a twin screw extruder wherein the resins are melted and kneaded, and extruding the resin material to produce polymer alloy pellets, which may be used for fabricating the catheter shaft.

The term polymer alloy used herein includes polymer blend, graft polymer, block copolymer, random copolymer, ion crosslinked polymer, interpenetrating polymer network (IPN), and the like. In the preparation of the polymer alloy, alloying agents and compatibilizing agents may be employed as desired. The polymer alloy may optionally include fillers and the like.

The catheter shaft of the present invention has a tensile modulus of up to 1,570 MPa (16,000 kg/cm$^2$).

The term, tensile modulus used herein is initial modulus or Young's modulus determined in accordance with, for example, JIS or ASTM by conventional tensile tests, wherein a hot pressed sheet is prepared from the pellets or from the catheter shaft, and tension stress is applied to the thus pressed sheet at room temperature and at a tensile rate of 1 mm/min.

To adjust the tensile modulus of the polymer material of up to 1,570 MPa (16,000 kg/cm$^2$), it would be preferable to employ an $\alpha$-olefin polymer of a low tensile modulus and an alloying polymer of a high tensile modulus.

The catheter shaft of the present invention has a pressure-resisting strength of at least 6.2 MPa (63 kg/cm$^2$). When the catheter shaft is used for a blood vessel dilatation catheter, it may preferably have a pressure-resisting strength of at least 11.1 MPa (113 kg/cm$^2$).

The pressure-resisting strength of the catheter shaft is determined in a pressure test wherein a fluid such as nitrogen gas is fed to the catheter shaft until it bursts. The pressure-resisting strength is calculated by the following equation known for a membrane :

$$\sigma_2 = P \times r/h.$$

In the above equation, $\sigma_2$ is tensile strength of the membrane, which is referred to as the pressure-resisting strength in the present invention; P is the pressure applied to the test sample upon bursting; r is the initial radius of the test sample; and h is the wall thickness of the test sample. The radius and the wall thickness of the test sample are determined by observing the cross section of the catheter shaft with a stereoscopic microscope. (See S. Timoshenko, "Strength of Materials", Part II, Second edition, page 165, D. van Nostrand Company Inc., New York, NY, 1941.)

As mentioned above, in the case of a blood vessel dilation catheter, the catheter shaft should endure a balloon inflation pressure of at least about 7 to 8 atmospheres. Therefore, the catheter shaft should have a burst pressure of at least 0.98 MPa (10 kg/cm$^2$) and in the catheter shaft of a practical size having an outer diameter of 1.07 mm, an inner diameter of 0.90 mm, and a wall thickness of 0.085 mm, this burst pressure corresponds to a pressure-resisting strength of at least 6.17 MPa (62.9 kg/cm$^2$). The catheter shaft may preferably have a pressure-resisting strength of at least 8.63 MPa (88 kg/cm$^2$) to endure a balloon inflation of 14 atmospheres. The catheter shaft may more preferably have a pressure-resisting strength of at least 11.1 MPa (113 kg/cm$^2$) which corresponds to a burst pressure of at least 1.77 MPa (18 kg/cm$^2$) for a catheter shaft of the above mentioned size. Such a highly burst-resistant catheter shaft of the present invention may be employed for a blood vessel dilatation catheter of high-pressure specification.

The catheter shaft of the present invention can be molded by conventional extrusion or injection molding. The catheter shaft may also be produced by multi-layer extrusion by employing the polymeric material of the present invention as the surface layer. It should be noted that the catheter shaft of the present invention can be molded at a relatively low temperature, and therefore, the molding of the present catheter shaft may be readily effected. It should also be noted that the material of the invention permits post-processing such as orientation.

The catheter shaft of the present invention is fabricated from a polymeric material which comprises an $\alpha$-olefin polymer, a polymer alloy of two or more $\alpha$-olefin polymers, or a polymer alloy of at least 10% by weight of an $\alpha$-olefin polymer with another polymer. Therefore, in producing the catheter shaft, the flexibility of the product may be readily controlled by varying the blend ratio of the polymeric material without using any plasticizing agent.

Since the catheter shaft of the present invention is fabricated from a polymeric material which comprises at least 10% by weight of an $\alpha$-olefin polymer having a tertiary carbon-hydrogen bond, the catheter shaft can be surface-modified by such means as plasma-induced graft polymerization to impart the surface of the catheter shaft with biocompatibility and/or blood compatibility.

The present invention is described in further detail by the following examples, which by no means limit the scope of the invention.

EXAMPLES

Comparative Example 1

A catheter shaft having an inner diameter of 0.9 mm, an outer diameter of 1.07 mm, and a wall thickness of 85 $\mu$m was produced with polypropylene (trade name, HIPOL, grade F401, manufactured by Mitsui Petrochemical Industries, Ltd.), which is an $\alpha$-olefin having a relatively high modulus. The polypropylene was used alone without blending with other resins.

Example 1

The polypropylene of the type used in Comparative Example 1 and polybutene-1 (trade name, BEAULON, grade P4000, manufactured by Mitsui Petrochemical Industries, Ltd.) having a tensile modulus of 422 MPa (4,300 kgf/cm$^2$) in a weight ratio of 80:20 were kneaded and extruded with a twin screw extruder to produce alloy pellets. A catheter shaft of the same size as Comparative Example 1, having an inner diameter of 0.9 mm, an outer diameter of 1.07 mm, and a wall thickness of 85 $\mu$m, was produced from the thus produced pellets.

Example 2

The procedure of Example 1 was repeated except that the weight ratio of the polypropylene to the polybutene-1 was 60:40.

Example 3

The procedure of Example 1 was repeated except that the weight ratio of the polypropylene to the polybutene-1 was 40:60.

Example 4

The procedure of Example 1 was repeated except that the weight ratio of the polypropylene to the polybutene-1 was 20:80.

Example 5

The procedure of Example 1 was repeated except that the weight ratio of the polypropylene to the polybutene-1 was 0:100.

Comparative Example 2

A catheter shaft of the same size as Example 1 was produced with a polyethylene (trade name, HI-ZEX, grade 3000B, manufactured by Mitsui Petrochemical Industries, Ltd.) This polyethylene has a modulus which is typical for the conventional resin material used for the shaft of the blood vessel dilatation catheter. The polyethylene was used alone without blending with other resins.

Comparative Example 3

The procedure of Comparative Example 2 was repeated except that the polyethylene used had a modulus lower than the one used in Comparative Example 2. The polyethylene used was of the trade name HI-ZEX, grade 5305E, manufactured by Mitsui Petrochemical Industries, Ltd.

Comparative Example 4

100 PHR by weight of straight polyvinyl chloride (S1001, manufactured by Kanegafuchi Chemical Industries Co., Ltd.), 55 PHR by weight of dioctylphthalate (manufactured by Kao K.K.), and a stabilizing agent were blended in a conventional manner and pelletized in an extruder. The thus produced pellets were used to produce a catheter shaft of the same size as Example 1.

Example 6

The polyethylene of the type used in Comparative Example 2 and polybutene-1 (trade name, BEAULON, grade M3110, manufactured by Mitsui Petrochemical Industries, Ltd.) in a weight ratio of 50:50 were kneaded and extruded with a twin screw extruder to produce alloy pellets. A catheter shaft of the same size as Example 1 was produced from the thus produced pellets.

Example 7

The polyethylene of the type used in Comparative Example 2 and 1,2-polybutadiene (trade name, JSR RB grade 810, manufactured by Japan Synthetic Rubber K.K.) in a weight ratio of 50:50 were kneaded and extruded with a twin screw extruder to produce alloy pellets. A catheter shaft of the same size as Example 1 was produced from the thus produced pellets.

Example 8

The polybutene-1 of the type used in Example 6 and the 1,2-polybutadiene of the type used in Example 7 in a weight ratio of 50:50 were kneaded and extruded with a twin screw extruder to produce alloy pellets. A catheter shaft of the same size as Example 1 was produced from the thus produced pellets.

Experiment 1

Hot-pressed sheets were prepared from the pellets used in Examples 1 to 8 and Comparative Examples 1 to 4, respectively, to evaluate the sheets for their tensile properties and adaptability to plasma treatment.

The tensile test was carried out at room temperature in accordance with ASTM D638 for a dumbell-shaped blanked sheet.

The adaptability to plasma treatment was evaluated by surface-treating the sheet by plasma-initiated graft polymerization in accordance with the method disclosed in Japanese Patent Application Kokai No. 2(1990)-263845, published as JP-A-41 41 046, namely, by irradiating the sheet with low-temperature plasma (Ar 0,13 $10^2$ Pa: (0.1 Torr) for 10 seconds, and thereafter introducing MEA (methoxyethyl acrylate) in gas phase under a reduced pressure to induce graft polymerization. The adaptability to plasma treatment was evaluated by observing the texture of the sample after wetting the sample to see whether the sample was lubricous or not. The results were evaluated to be good when the surface became lubricous.

Table 1

| | Composition | Breaking strength, MPa | (kgf/cm$^2$) | Initial modulus, MPa | (kgf/cm$^2$) | Adaptability to plasma treatment |
|---|---|---|---|---|---|---|
| Comparative Example 1 | PP/PB 100/0 | 38.1 | 388 | 1,913 | 19,500 | good |
| Example 1 | PP/PB 80/20 | 37.7 | 384 | 1,501 | 15,300 | good |
| Example 2 | PP/PB 60/40 | 38.5 | 392 | 1,118 | 11,400 | good |
| Example 3 | PP/PB 40/60 | 39.9 | 407 | 877 | 8,940 | good |
| Example 4 | PP/PB 20/80 | 37.2 | 379 | 691 | 7,040 | good |
| Example 5 | PP/PB 0/100 | 36.7 | 374 | 437 | 4,450 | good |
| Comparative Example 2 | PE 3000B | 30.6 | 312 | 1,580 | 16,100 | poor |
| Comparative Example 3 | PE 5305E | 24.5 | 250 | 1,275 | 13,000 | poor |
| Comparative Example 4 | PVC | 20 | 204 | 15.7 | 160 | good |
| Example 6 | PE/PB 50/50 | 36.3 | 370 | 932 | 9,500 | good |
| Example 7 | PE/RB 50/50 | 19.4 | 198 | 874 | 8,900 | good |
| Example 8 | PB/RB 50/50 | 25.4 | 259 | 143 | 1,460 | good |
| PP: polypropylene; PB: polybutene-1; PE: polyethylene; PVC: polyvinyl chloride; RB: 1,2-polybutadiene | | | | | | |

From a clinical point of view, the catheter shaft may preferably have a modulus of not more than 1,570 MPa (16,000 kg/cm$^2$). The polypropylene of Comparative Example 1 and the polyethylene of Comparative Example 2 were, therefore, undesirable.

In the case of polypropylene/polybutene-1 system of Examples 1 to 5 and polyethylene/polybutent-1 system of Example 6, the flexibility (initial modulus) of the sheet could be widely controlled by changing the ratio of the polybutene-1 to the polypropylene or the polyethylene without inducing any significant change in the breaking strength. The flexibility of the sheet was thus. controllable.

In the case of PE/PB, and PE/RB systems of Examples 6 and 7, the bad flexibility of polyethylene as shown in the comparative example 2 was improved by adding PB or RB.

In the case of PB/RB system of Example 8, the flexibility was excellent as rubbers.

On the other hand, in the case of Comparative Examples 2 and 3 wherein the polyethylene was used, the decrease in the modulus was accompanied with a decrease in the breaking strength. In the case of Comparative Example 4 wherein polyvinyl chloride was used with other additives, the breaking strength was far from being sufficient.

It is generally known that an addition of a plasticizing agent and change in the degree of polymerization or structure involve a decrease in both the strength and the modulus. Also, a decrease in the modulus is usually accompanied with a decrease in the strength of the material, as demonstrated in Comparative Examples 2 and 3. Therefore, it is unusually favorable that the polypropylene/polybutene-1 and polyethylene/polybutene-1 systems have retained their strength over a wide range of blending ratios to enable stable control of flexibility without any limitation imposed by other physical properties.

The adaptability to plasma treatment evaluated by surface-treating the sheet by plasma-initiated graft polymerization was also superior in the Examples of the present invention compared to Comparative Examples 2 and 3 wherein polyethylene was used. It is estimated that such a superiority of the Examples of the invention is due to relatively easily induced hydrogen atom removal owing to tertiary carbons present in the polybutene-1 or its alloy with the polypropylene or the polyethylene in contrast to the polyethylene which solely comprises secondary carbons because of its structure. Therefore, the polymeric materials of the present invention are superior to the polyethylene in imparting the anti-thrombotic properties and lubricity to the material surface.

Experiment 2

The catheter shafts prepared in Examples 1 to 8 and Comparative Examples 1 to 4 were evaluated for their burst pressure in a pressure test wherein nitrogen gas was fed into the shaft. Pressure-resisting strength of the catheter shaft was calculated from the thus evaluated burst pressure by the following equation. The results are shown in Table 2.

$$\sigma_2 = pb \times 1.07/2/0.085$$

$\sigma_2$: pressure-resisting strength,
pb: burst pressure,
size of the catheter shaft:

outer diameter: 1.07 mm,
inner diameter: 0.90 mm,
wall thickness: 0.085 mm.

The catheter shafts were surface-treated by plasma-initiated graft polymerization in the same manner as Experiment 1, and then treated in the manner described in Japanese Patent Publication No. 1(1989)-33181 to covalently link maleic anhydride-based high polymers on their surface. The treatment was effected to modify the catheter shaft surfaces so that they may become lubricous under moist conditions. The results were evaluated to be good when the surfaces became lubricous after wetting.

Table 2

| | Composition | Burst pressure, MPa | (kgf/cm$^2$) | Pressure-resisting strength, MPa | (kgf/cm$^2$) | Adaptability to surface treatment |
|---|---|---|---|---|---|---|
| Comparative Example 1 | PP/PB 100/0 | 2.35 | 24 | 14.8 | 151 | good |
| Example 1 | PP/PB 80/20 | 2.55 | 26 | 16 | 163 | good |
| Example 2 | PP/PB 60/40 | 2.35 | 24 | 14.8 | 151 | good |
| Example 3 | PP/PB 40/60 | 2.35 | 24 | 14.8 | 151 | good |
| Example 4 | PP/PB 20/80 | 2.35 | 24 | 14.8 | 151 | good |
| Example 5 | PP/PB 0/100 | 2.55 | 26 | 16 | 163 | good |
| Comparative Example 2 | PE 3000B | 2.06 | 21 | 13 | 132 | poor |
| Comparative Example 3 | PE 5305E | 1.77 | 18 | 11.09 | 113 | poor |
| Comparative Example 4 | PVC | 0.88 | 9 | 5.6 | 57 | good |
| Example 6 | PE/PB 50/50 | 2.45 | 25 | 15.4 | 157 | good |

Table 2 (continued)

| | Composition | Burst pressure, MPa | (kgf/cm$^2$) | Pressure-resisting strength, MPa | (kgf/cm$^2$) | Adaptability to surface treatment |
|---|---|---|---|---|---|---|
| Example 7 | PE/RB 50/50 | 1.37 | 14 | 8.7 | 88 | good |
| Example 8 | PB/RB 50/50 | 1.77 | 18 | 11.09 | 113 | good |
| PP: polypropylene; PB: polybutene-1; PE: polyethylene; PVC: polyvinyl chloride; RB: 1,2-polybutadiene | | | | | | |

As mentioned above, a catheter shaft is required to have sufficient mechanical strength, and in particular, pressure-resisting strength. In conventional catheter shafts, improvement in flexibility has been attained at the sacrifice of the pressure-resisting strength. In contrast, the flexibility of the catheter shaft of the present invention is controlled over a wide range without detracting from the pressure-resisting strength.

Pressure-resisting strength of the catheter may be increased by thickening the wall of the catheter shaft, although such a thickening of the wall at the sacrifice of the size of the lumen is undesirable. The lumen of the catheter shaft should be sufficiently large to thereby decrease the flow resistance of the fluid flowing therethrough and secure smooth inflation and deflation of the balloon. Therefore, the wall of the catheter shaft inevitably becomes thin.

As mentioned above, in the case of a blood vessel dilatation catheter, the catheter shaft should endure a balloon inflation pressure of at least about 7 to 8 atmospheres. Therefore, the catheter shaft should have a burst pressure of at least 0.98 MPa (10 kg/cm$^2$), and in the catheter shaft of a practical side having an outer diameter of 1.07 mm, an inner diameter of 0.90 mm, and a wall thickness of 0.085 mm, this burst pressure corresponds to a pressure-resisting strength of at least 6.17 MPa (62.9 kg/cm$^2$). The catheter shaft may preferably have a pressure-resisting strength of at least 88 kg/cm$^2$ to endure a balloon inflation at 14 atmospheres. The catheter shaft may more preferably have a pressure-resisting strength of at least 11.1 MPa (113 kg/cm$^2$) which corresponds to a burst pressure of at least 1.77 MPa (18 kg/cm$^2$) for the catheter shaft of the above mentioned size. Such a highly burst-resistant catheter shaft of the present invention may be advantageously employed for a blood vessel dilatation catheter of high-pressure specification.

The data shown in Table 2 construed with the data shown in Table 1 reveal that, in the case of the polypropylene/polybutene-1 system of Examples 1 to 5 and the polyethylene/polybutene-1 system of Example 6, no decrease in the burst pressure and pressure-resisting strength is induced with the change in the flexibility (initial modulus) of the catheter shaft. Such results are consistent with the results of the tensile test in Experiment 1.

Moreover, the burst pressure of the catheter shafts of Examples 1 to 6 was 2.35 MPa (24 kg/cm$^2$) or higher, indicating that they could resist a balloon inflation pressure of as high as 20 atmospheres or even higher. Such highly pressure-resistant catheter shafts may be employed for the dilatation of highly constricted blood vessel, such as tight, rigid stenosis.

The catheter shafts of Examples 7 and 8 had a burst pressure higher than 0.98 MPa (10 kg/cm$^2$), namely a pressure-resisting strength higher than 6.2 MPa (63 kg/cm$^2$). Therefore, the catheter shafts of Examples 7 and 8 can fully endure 7 to 8 atmospheres, which is the pressure typically applied upon inflation of the balloon.

Such capability of varying the flexibility with no significant decrease in the pressure-resisting strength is a remarkable improvement over conventional catheter materials in which increase in flexibility was accompanied with a significant decrease in the pressure-resisting strength.

With regard to the adaptability to surface-treatment, polymeric materials of the Examples of the present invention, polypropylene of Comparative Example 1, and polyvinyl chloride of Comparative Example 4 exhibited good lubricity under moist conditions, while the polyethylene materials of Comparative Examples 2 and 3 exhibited poor lubricity under moist conditions. Such a poor lubricity of the polyethylene under moist conditions is due to poor adaptability to plasma treatment of the polyethylene, which in turn resulted in difficulty of covalently linking the maleic anhydride-based high polymer on the surface. The surface lubricity under moist conditions is improved in the polyethylene/polybutene-1 and the polyethylene/1,2-polybutadiene alloys of Examples 6 and 7. Such an improvement in wet lubricity is estimated to have been introduced by alloying with the α-olefin polymer having a tertiary carbon-hydrogen bond. In other words, the surface properties of α-olefin, a material which inherently has a low surface abrasiveness, could be further improved by alloying with the α-olefin polymer having a tertiary carbon-hydrogen bond to acquire wet-lubricity. Such adaptability to coating with a hydrophilic high polymer enables surface treatment to impart anti-thrombotic properties to the material.

As described above, the catheter shaft of the present invention is fabricated from a mass-produced, inexpensive, safe polymeric material which comprises an α-olefin polymer, a polymer alloy of two or more α-olefin polymers, or a polymer alloy of at least 10% by weight of an α-olefin polymer with another polymer. Therefore, the flexibility of the catheter shaft can be controlled without using any hazardous plasticizing agent. In addition, since the catheter shaft comprises such polymeric material, no substance is likely to be eluted out of the catheter shaft, while no agent is likely to be adsorbed on the catheter shaft. Such polymeric material also has excellent chemical resistance, heat resistance, creep properties, and storage stability. Furthermore, the polymeric material of the present invention has an adequate melting point, and therefore, the catheter shaft can be readily heat-sealed to enable a convenient assembly of the catheter.

The most significant merits of the catheter shaft of the present invention, however, is the capability of varying the flexibility over a wide range without detracting from the strength by selecting adequate combination and blending ratio of the polymer components, and the capability of remarkably improving the biocompatibility and/or the blood compatibility by surface modification.

Since the catheter shaft of the present invention has a remarkably high pressure-resisting strength in combination with sufficient flexibility, it may be used for the thin-walled shaft portion of a blood vessel dilatation catheter, wherein a lumen of a sufficient size is defined within the small-diameter shaft to allow for a smooth flow of the balloon-inflation liquid therethrough. More illustratively, the catheter shaft of the present invention can fully endure a pressure of 0.71 to 0.81 MPa (7 to 8 atmospheres), which is the pressure typically applied upon balloon inflation. When the catheter shaft is fabricated from a polypropylene/polybutene-1 polymer alloy or a polyethylene/polybutene-1 polymer alloy, it can endure a pressure of as high as 20 atmospheres or even higher so that it can be used for a blood dilatation catheter used for a highly constricted blood vessel as in the case of tight, rigid stenosis, which is the case requiring an exceptionally high pressure for the dilatation.

In addition, since the flexibility, and hence, the trackability of the catheter shaft of the present invention can be adjusted without detracting from its strength by changing the composition of the polymeric material, the shaft diameter can be reduced throughout its length without detracting from operability, in particular, pushability of the catheter shaft. Such a small-diameter catheter shaft having sufficient trackability can be used for securing blood flow through the coronary artery.

One skilled in the art will appreciate from a reading of this disclosure that various changes and modifications can be made without departing from the scope of the invention. In this regard, one skilled in the art will appreciate that the physical properties of the catheter shaft can be even more widely varied by changing the type of the polymer used, for example, by replacing the polypropylene used in the Examples with ethylene-propylene copolymer, or by replacing the polybutene-1 used in the Examples with polybutene-1 of the grade having a lower modulus.

## Claims

1. A catheter shaft fabricated from a polymeric material comprising an $\alpha$-olefin polymer, characterised in that said polymeric material comprises at least 10% by weight of an $\alpha$-olefin polymer selected from the group consisting of polybutene, polyisoprene, polyisobutylene, propylene-butene copolymers and copolymers of poly-4-methyl-1-pentene, the rest of the polymeric material being another polymer selected from the group consisting of polyethylene, ethylene-vinyl acetate copolymers, ethylene-vinyl chloride copolymers, polypropylene, ethylene-propylene copolymers, and propylene-butene copolymers, said catheter shaft having a tensile modulus of up to 1,570 MPa (16,000 kg/cm$^2$) and a pressure-resisting strength of at least 6.2 MPa (63 kg/cm$^2$).

2. The catheter shaft according to claim 1, wherein said $\alpha$-olefin polymer is the polybutene-1.

3. The catheter shaft according to any one of claims 1 and 2 wherein said catheter shaft is the shaft of a blood vessel-dilating catheter.

4. A blood vessel-dilating catheter shaft having a lumen through which a highly pressurized dilation fluid is introduced to inflate a tip balloon to thereby dilate stenosis located in a blood vessel of a patient, said catheter shaft being fabricated from a polymeric material and characterised by having a tensile modulus of up to 1,570 MPa (16,000 kg/cm$^2$) and a pressure-resisting strength of at least 6.2 MPa (63 kg/cm$^2$), said polymeric material consisting essentially of:

   20 to 80% by weight of an $\alpha$-olefin polymer selected from the group consisting of polybutene, 1,2-polybutadiene, polyisoprene, polyisobutylene, propylene-butene copolymers and copolymers of poly-4-methyl-1-pentene; and

   80 to 20% by weight of another polymer selected from the group consisting of polyethylene, ethylene-vinyl acetate copolymers, ethylene-vinyl chloride copolymers, polypropylene, ethylene-propylene copolymers and propylene-butene copolymers.

5. The catheter shaft according to claim 4, wherein said $\alpha$-olefin polymer is the polybutene-1 or the 1,2-polybutadiene.

6.  The catheter shaft according to any one of claims 1 to 5, wherein said α-olefin polymer has a tensile modulus of up to 491 MPa (5,000 kg/cm$^2$).

7.  The catheter shaft according to any one of claims 1 to 6, wherein said other polymer is at least one member selected from polypropylene and polyethylene.

8.  The catheter shaft according to any one of claims 1 to 7, wherein said α-olefin polymer is polybutene-1, and said other polymer is polypropylene.

9.  The catheter shaft according to any one of claims 1 to 8, wherein the pressure-resisting strength is at least 11.1 MPa (113 kg/cm$^2$).

10. The catheter shaft according to any one of claims 1 to 9, wherein said polymer material is surface-modified to impart the material with biocompatibility.

11. The catheter shaft according to claim 10, wherein the polymer material has been subjected to plasma treatment followed by coating with a maleic anhydride-based polymeric composition.

12. The catheter shaft according to claim 11, wherein the polymer material has been subjected to plasma-initiated graft polymerization with an acrylate compound.

**Patentansprüche**

1.  Kathederschaft, hergestellt aus einem ein α-Olefinpolymer umfassenden Polymermaterial,
    **dadurch gekennzeichnet, daß**
    das Polymermaterial mindestens 10 Gew.-% eines α-Olefinpolymers umfaßt, das aus der aus Polybuten, Polyisopren, Polyisobutylen, Propylen-Buten-Copolymeren und Copolymeren von Poly-4-methyl-1-penten bestehenden Gruppe ausgewählt ist, und der Rest des Polymermaterials ein weiteres Polymer ist, das aus der aus Polyethylen, Ethylen-Vinylacetat-Copolymeren, Ethylen-Vinylchlorid-Copolymeren, Polypropylen, Ethylen-Propylen-Copolymeren und Propylen-Buten-Copolymeren bestehenden Gruppe ausgewählt ist, wobei der Kathederschaft ein Zugmodul von bis zu 1570 MPa (16000 kg/cm$^2$) sowie eine Druckwiderstandsfestigkeit von mindestens 6,2 MPa (63 kg/cm$^2$) besitzt.

2.  Kathederschaft nach Anspruch 1, wobei das α-Olefinpolymer Poly-1-buten ist.

3.  Kathederschaft nach einem der Ansprüche 1 und 2, wobei der Kathederschaft der Schaft eines blutgefäßerweiternden Katheders ist.

4.  Blutgefäßerweiternder Katheder mit einem Lumen, durch den eine unter hohem Druck stehende erweiternde Flüssigkeit zum Aufblasen eines an der Spitze befindlichen Ballons eingeleitet wird, um dadurch eine in einem Blutgefäß eines Patienten befindliche Stenose zu erweitern, wobei der Kathederschaft aus einem Polymermaterial gefertigt und durch ein Zugmodul von bis zu 1570 MPa (16000 kg/cm$^2$) sowie eine Druckwiderstandsfestigkeit von mindestens 6,2 MPa (63 kg/cm$^2$) ausgezeichnet ist und das Polymermaterial im wesentlichen besteht aus:

    20 bis 80 Gew.-% eines α-Olefinpolymers, das aus der aus Polybuten, 1,2-Polybutadien, Polyisopren, Polyisobutylen, Propylen-Buten-Copolymeren und Copolymeren von Poly-4-methyl-1-penten bestehenden Gruppe ausgewählt ist; und
    80 bis 20 Gew.-% eines weiteren Polymers, das aus der aus Polyethylen, Ethylen-Vinylacetat-Copolymeren, Ethylen-Vinylchlorid-Copolymeren, Polypropylen, Ethylen-Propylen-Copolymeren und Propylen-Buten-Copolymeren bestehenden Gruppe ausgewählt ist.

5.  Kathederschaft nach Anspruch 4, wobei das α-Olefinpolymer Poly-1-buten oder 1,2-Polybutadien ist.

6.  Kathederschaft nach einem der Ansprüche 1 bis 5, wobei das α-Olefinpolymer ein Zugmodul von bis zu 491 MPa (5000 kg/cm$^2$) besitzt.

7.  Kathederschaft nach einem der Ansprüche 1 bis 6, wobei das weitere Polymer mindestens eine aus Polypropylen und Polyethylen ausgewählte Verbindung ist.

8. Kathederschaft nach einem der Ansprüche 1 bis 7, wobei das $\alpha$-Olefinpolymer Poly-1-buten und das weitere Polymer Polypropylen ist.

9. Kathederschaft nach einem der Ansprüche 1 bis 8, wobei die Druckwiderstandsfestigkeit mindestens 11,1 MPa (113 kg/cm$^2$) beträgt.

10. Kathederschaft nach einem der Ansprüche 1 bis 9, wobei das Polymermaterial oberflächenmodifiziert ist, um dem Material Biokompatibilität zu verleihen.

11. Kathederschaft nach Anspruch 10, wobei das Polymermaterial einer Plasmabehandlung, gefolgt von einer Beschichtung mit einer Polymer-Zusammensetzung auf Maleinsäureanhydridbasis, unterworfen wurde.

12. Kathederschaft nach Anspruch 11, wobei das Polymermaterial einer Plasma-ausgelösten Pfropfpolymerisation mit einer Acrylat-Verbindung unterworfen wurde.

## Revendications

1. Tige de cathéter fabriquée à partir d'une matière polymérique comprenant un polymère d'$\alpha$-oléfine, caractérisée en ce que ladite matière polymérique comprend au moins 10 % en masse d'un polymère d'$\alpha$-oléfine choisi dans le groupe consistant en le polybutène, le polyisoprène, le polyisobutylène, les copolymères propylène-butène et les copolymères de poly-4-méthylpent-1-ène, le reste de la matière polymérique étant un autre polymère choisi dans le groupe consistant en le polyéthylène, les copolymères éthylène-acétate de vinyle, les copolymères éthylène-chlorure de vinyle, le polypropylène, les copolymères éthylène-propylène et les copolymères propylène-butène, ladite tige de cathéter ayant un module de traction allant jusqu'à 1 570 MPa (16 000 kg/cm$^2$) et une résistance à la pression d'au moins 6,2 MPa (63 kg/cm$^2$).

2. Tige de cathéter selon la revendication 1, dans laquelle ledit polymère d'$\alpha$-oléfine est le polybut-1-ène.

3. Tige de cathéter selon l'une quelconque des revendications 1 et 2, où ladite tige de cathéter est la tige d'un cathéter de dilatation de vaisseaux sanguins.

4. Tige de cathéter de dilatation de vaisseaux sanguins ayant une lumière dans laquelle est introduit un fluide de dilatation sous haute pression pour gonfler un ballonnet d'extrémité pour dilater ainsi une sténose située dans un vaisseau sanguin d'un patient, ladite tige de cathéter étant fabriquée à partir d'une matière polymérique et étant caractérisée en ce qu'elle a un module de traction allant jusqu'à 1 570 MPa (16 000 kg/cm$^2$) et une résistance à la pression d'au moins 6,2 MPa (63 kg/cm$^2$), ladite matière polymérique consistant essentiellement en :

   20 à 80 % en masse d'un polymère d'$\alpha$-oléfine choisi dans le groupe consistant en le polybutène, le polybuta-1,2-diène, le polyisoprène, le polyisobutylène, les copolymères propylène-butène et les copolymères de poly-4-méthylpent-1-ène ; et
   80 à 20 % en masse d'un autre polymère choisi dans le groupe consistant en le polyéthylène, les copolymères éthylène-acétate de vinyle, les copolymères éthylène-chlorure de vinyle, le polypropylène, les copolymères éthylène-propylène et les copolymères propylène-butène.

5. Tige de cathéter selon la revendication 4, dans laquelle ledit polymère d'$\alpha$-oléfine est le polybut-1-ène ou le polybuta-1,2-diène.

6. Tige de cathéter selon l'une quelconque des revendications 1 à 5, dans laquelle ledit polymère d'$\alpha$-oléfine a un module de traction allant jusqu'à 491 MPa (5 000 kg/cm$^2$).

7. Tige de cathéter selon l'une quelconque des revendications 1 à 6, dans laquelle ledit autre polymère est au moins un élément choisi parmi le polypropylène et le polyéthylène.

8. Tige de cathéter selon l'une quelconque des revendications 1 à 7, dans laquelle ledit polymère d'$\alpha$-oléfine est le polybut-1-ène et ledit autre polymère est le polypropylène.

9. Tige de cathéter selon l'une quelconque des revendications 1 à 8, dans laquelle la résistance à la pression est d'au moins 11,1 MPa (113 kg/cm$^2$).

**10.** Tige de cathéter selon l'une quelconque des revendications 1 à 9, dans laquelle ladite matière polymérique est modifiée en surface pour conférer à la matière une biocompatibilité.

**11.** Tige de cathéter selon la revendication 10, dans laquelle la matière polymérique a été soumise à un traitement par plasma suivi par un revêtement avec une composition polymérique à base d'anhydride maléique.

**12.** Tige de cathéter selon la revendication 11, dans laquelle la matière polymérique a été soumise à une polymérisation par greffage initiée par plasma avec un composé d'acrylate.